# EUROPÄISCHE PATENTSCHRIFT

(11) **EP 3 960 407 B1**
(45) Veröffentlichungstag und Bekanntmachung des Hinweises auf die Patenterteilung: **29.10.2025**
(21) Anmeldenummer: 21192093.9
(22) Anmeldetag: 19.08.2021
(51) Int. Cl.: B27D 5/00, B27C 5/00

(54) **VERFAHREN ZUM POSITIONIEREN EINES BEARBEITUNGSAGGREGATS ZU EINEM ZU BEARBEITENDEN WERKSTÜCKS SOWIE BEARBEITUNGSEINRICHTUNG**
MACHINING DEVICE AND METHOD FOR POSITIONING A MACHINING UNIT TO A WORKPIECE TO BE MACHINED
PROCÉDÉ DE POSITIONNEMENT D'UNE UNITÉ D'USINAGE SUR UNE PIÈCE, AINSI QU'AGENCEMENT D'USINAGE

(30) Priorität: 20.08.2020 DE 102020121862
(43) Veröffentlichungstag der Anmeldung: 02.03.2022
(73) Patentinhaber: HOMAG GmbH, 72296 Schopfloch (DE)
(72) Erfinder: Kalmbach, Wilhelm, 72296 Schopfloch (DE)
(74) Vertreter: Hoffmann Eitle

(56) Entgegenhaltungen:
- EP-A1- 2 191 948
- EP-A1- 3 575 052
- DE-A1- 3 029 433
- DE-C1- 10 124 307

## Beschreibung

### Technisches Gebiet

Die Erfindung betrifft ein Verfahren zum Positionieren eines Bearbeitungsaggregats zu einem zu bearbeitenden Werkstück gemäß dem Oberbegriff des Anspruchs 1, sowie eine Bearbeitungseinrichtung zum Bearbeiten von Werkstücken gemäß dem Oberbegriff des Anspruchs 9. Ein solches Verfahren und eine solche Bearbeitungseinrichtung sind aus dem Dokument DE 30 29 433 A1.

### Stand der Technik

Es sind Bearbeitungseinrichtungen zum Bearbeiten von Werkstücken bekannt, insbesondere Holzbearbeitungseinrichtungen, die sowohl als stationäre Bearbeitungsmaschine oder auch als Durchlaufmaschine, bei welcher die Werkstücke während des Bearbeitungsvorgangs in einer Durchlaufrichtung relativ zu einem Bearbeitungswerkzeug bewegt werden, ausgebildet sind. Dem Bearbeitungswerkzeug kann eine Tasteinrichtung zugeordnet sein, wobei eine oder mehrere Tastrollen der Tasteinrichtung auf einer Werkstückoberfläche abrollen, um während des Bearbeitungsvorgangs eine Führung für das Bearbeitungswerkzeug zu bilden. Zum Ausführen des Tastvorgangs fährt das sich in Durchlaufrichtung bewegende Werkstück gegen die Tastrolle, sodass eine Werkstückvorderkante die Tastrolle und somit das gesamte Bearbeitungsaggregat in eine Tastposition drückt. Durch den Kontakt des Werkstücks mit der Tastrolle tritt ein Stoßimpuls auf, der das Bearbeitungsaggregat und somit auch das Bearbeitungswerkzeug in einen Schwingungszustand versetzt. Insbesondere im Anfangsbereich des Bearbeitungsvorgangs bewirkt dieser Schwingungszustand eine Beeinträchtigung der Bearbeitungsqualität.

Es ist das Dokument DE 30 29 433 A1 bekannt, das eine doppelseitige Umleimerfräse mit Dreiseitentastung zeigt.

Im Dokument DE 101 24 307 C1 wird ein Kantenfräsaggregat für eine programmgesteuerte Durchlaufmaschine beschrieben.

### Darstellung der Erfindung

Der Erfindung liegt die Aufgabe zugrunde, ein Verfahren vorzuschlagen, durch welches eine verbesserte Bearbeitungsqualität bei einem Bearbeitungsvorgang mit einem Bearbeitungsaggregat an einem Werkstück erreicht wird. Zudem ist es Aufgabe der Erfindung eine Bearbeitungseinrichtung zum Bearbeiten von Werkstücken vorzuschlagen, welches eine verbesserte Bearbeitungsqualität ermöglicht.

Ein Verfahren zum Positionieren eines Bearbeitungsaggregats zu einem zu bearbeitenden Werkstück ist in Anspruch 1 definiert. Eine Bearbeitungseinrichtung zum Bearbeiten von Werkstücken ist in Anspruch 9 definiert. Unteransprüche beziehen sich auf bestimmte Ausführungsformen.

Die Aufgabe wird durch ein Verfahren zum Positionieren eines Bearbeitungsaggregats zu einem zu bearbeitenden Werkstück, bei dem zwischen dem Bearbeitungsaggregat und dem zu bearbeitenden Werkstück eine Relativbewegung ausgeführt wird, eine Tasteinrichtung des Bearbeitungsaggregats zum Abtasten einer Werkstückoberfläche in Kontakt mit dem Werkstück gebracht wird und mittels der Tasteinrichtung ein geführter Bearbeitungsvorgang durch ein Bearbeitungswerkzeug des Bearbeitungsaggregats ausgeführt wird, gelöst, wobei das Bearbeitungsaggregat vor dem Kontakt mit dem Werkstück in einer Bereitschaftsposition angeordnet wird, in der wenigstens ein Tastelement der Tasteinrichtung in Bezug zum Werkstück oberhalb oder unterhalb einer imaginären Werkstückebene, die in der abzutastenden Werkstückoberfläche liegt, angeordnet ist, und das wenigstens eine Tastelement in die Tastposition oder eine Zwischenposition überführt wird, indem durch eine Stelleinrichtung eine zur imaginären Werkstückebene gerichtete Stellbewegung des Bearbeitungsaggregats angesteuert wird.

Durch ein solches Verfahren ist es ermöglicht, das wenigstens eine Tastelement der Tasteinrichtung in Kontakt zum Werkstück zu bringen, ohne einen nennenswerten Stoßimpuls auf das Bearbeitungsaggregat zu übertragen. Auf diese Weise kann verhindert werden, dass das Bearbeitungsaggregat in einen Schwingungszustand versetzt wird, der eine Beeinträchtigung der Bearbeitungsqualität insbesondere im Anfangsbereich des Bearbeitungsvorgangs bewirken würde. Das Bearbeitungsaggregat ist insbesondere für eine Bearbeitungseinrichtung vorgesehen. Bei der Bearbeitungseinrichtung kann es sich bevorzugt um eine Holzbearbeitungseinrichtung handeln. Die zu bearbeitenden Werkstücke sind bevorzugt Werkstücke, die zumindest teilweise aus Holz, Holzwerkstoffen, Kunststoff, Verbundwerkstoff oder dergleichen ausgebildet sind.

Eine bevorzugte Weiterbildung des Verfahrens kann vorsehen, dass die Stellbewegung des Bearbeitungsaggregats aus der Bereitschaftsposition in die Tastposition oder die Zwischenposition einen Stellweg aufweist, der eine Länge von etwa 2,0 mm oder kürzer, vorzugsweise etwa 1,0 mm oder kürzer, aufweist.

Durch einen solchen kurzen Stellweg kann eine sehr kurze Stellbewegung des Bearbeitungsaggregats ausgebildet werden. Dadurch kann eine signifikante Verringerung des Stoßimpulses zwischen dem Bearbeitungsaggregat und dem Werkstück erreicht werden. Der Schwingungszustand des Bearbeitungsaggregats kann dadurch nahezu eliminiert werden, wodurch mit dem Bearbeitungswerkzeug eine besonders hohe Bearbeitungsqualität erreichbar ist.

Eine vorteilhafte Ausgestaltung des Verfahrens kann zudem vorsehen, dass die Stellbewegung des Bearbeitungsaggregats als eine lineare oder nichtlineare Stellbewegung ausgeführt wird. Zudem kann die Stellbewegung des Bearbeitungsaggregats im Wesentlichen orthogonal zur abzutastenden Werkstückoberfläche ausgeführt werden. Auf diese Weise kann die Stellbewegung des Bearbeitungsaggregats unmittelbar zum Werkstück gerichtet ausgeführt werden, d.h. auf einem direkten Weg, sodass ein kurzer Stellweg ausgebildet werden kann.

In einer bevorzugten Ausgestaltung des Verfahrens kann vorgesehen sein, dass ein als Tastrolle ausgebildetes Tastelement mit einer Tastfläche auf die Werkstückoberfläche aufgesetzt wird, indem die Stellbewegung des Bearbeitungsaggregats aus der Bereitschaftsposition in die Tastposition angesteuert wird, nachdem eine in Vorschubrichtung gesehene Vorderkante des Werkstücks eine imaginäre Tastgrenze, die durch die Mittelachse der Tastrolle verläuft und die orthogonal zur Werkstückoberfläche ausgerichtet ist, passiert hat oder die Stellbewegung zum Zeitpunkt des Passierens der imaginären Tastgrenze ausgeführt wird.

Indem das Tastelement auf die Werkstückoberfläche aufgesetzt wird, kann vermieden werden, dass das Werkstück durch die Vorschubbewegung gegen das Tastelement bewegt wird und dieses in die Tastposition drückt. Auf diese Weise kann der Stoßimpuls durch das Werkstück auf das Bearbeitungsaggregat vollständig vermieden werden. Durch eine entsprechende Ansteuerung der Stellbewegung kann zudem ein sanftes Aufsetzen des Tastelements auf die Werkstückoberfläche ermöglicht sein.

Eine weitere Ausgestaltung des Verfahrens kann zudem vorsehen, dass die Stellbewegung des Bearbeitungsaggregats aus der Bereitschaftsposition in die Zwischenposition angesteuert wird, bevor eine in Vorschubrichtung gesehene Vorderkante des Werkstücks eine imaginäre Tastgrenze, die durch die Mittelachse eines als Tastrolle ausgebildeten Tastelements verläuft und die orthogonal zur Werkstückoberfläche ausgerichtet ist, passiert hat, wobei eine Tastfläche des Tastelements in der Zwischenposition in Bezug zum Werkstück unterhalb der imaginären Werkstückebene angeordnet ist und das Tastelement durch die Vorschubbewegung des Werkstücks durch einen Tasthub in die Tastposition gedrückt wird.

Indem das Tastelement durch das Werkstück in die Tastposition gedrückt wird kann sichergestellt werden, dass die Werkstückoberfläche bereits ab der Vorderkante des Werkstücks abgetastet wird. Auf diese Weise kann die Tasteinrichtung des Bearbeitungsaggregats mit nur einem Tastelement ausgebildet werden.

In einer vorteilhaften Weiterbildung des Verfahrens kann der Tasthub aus der Zwischenposition in die Tastposition einen Hubweg von weniger als etwa 1,0 mm, vorzugsweise weniger als etwa 0,5 mm, aufweisen.

Besonders bevorzugt kann der Tasthub nur wenige zehntel Millimeter, beispielsweise etwa 0,1 mm bis 0,3 mm, sein. Durch einen solchen kurzen Hubweg aus der Zwischenposition in die Tastposition kann erreicht werden, dass das Werkstück beim Kontakt mit dem Bearbeitungsaggregat einen besonders geringen Stoßimpuls überträgt. Obwohl das Bearbeitungsaggregat in die Tastposition gedrückt wird, wird das Bearbeitungsaggregat in keinen Schwingungszustand versetzt.

In einer weiteren vorteilhaften Ausgestaltung des Verfahrens kann zudem vorgesehen sein, dass die Stellbewegung in die Zwischenposition ausgeführt wird, wenn der Abstand der Werkstückvorderkante des sich in Vorschubrichtung bewegenden Werkstücks zur imaginären Tastgrenze 10 mm oder weniger ist, vorzugsweise 5 mm oder weniger.

Durch diesen Abstand, d.h. dem frühzeitigen Ansteuern der Stellbewegung, kann sichergestellt werden, dass das Tastelement nicht bereits vor dem Überführen in die Zwischenposition bzw. während dem Ausführen der Stellbewegung in Kontakt mit dem Werkstück kommt.

Die Aufgabe wird zudem durch eine Bearbeitungseinrichtung gemäß Anspruch 9 gelöst.

Dadurch kann das Bearbeitungsaggregat durch die Stelleinrichtung zumindest zwischen einer Bereitschaftsposition, in der wenigstens ein Tastelement der Tasteinrichtung in Bezug zum Werkstück oberhalb einer imaginären Werkstückebene, die in einer abzutastenden Werkstückoberfläche liegt, und einer Tastposition, in der das wenigstens eine Tastelement in Kontakt zu der abzutastenden Werkstückoberfläche gebracht ist, überführbar sein, wobei durch die Stelleinrichtung eine zur Werkstückoberfläche gerichtete Stellbewegung des Bearbeitungsaggregats ansteuerbar ist. Auf diese Weise kann verhindert werden, dass durch die Relativbewegung zwischen dem Werkstück und dem Bearbeitungsaggregat ein signifikanter Stoßimpuls auf das Bearbeitungsaggregat übertragen wird, wenn das Tastelement der Tasteinrichtung in Kontakt mit dem Werkstück gebracht wird. Das Bearbeitungsaggregat wird dadurch in keinen nennenswerten Schwingungszustand versetzt, sodass keine Beeinträchtigung der Bearbeitungsqualität insbesondere im Anfangsbereich des Bearbeitungsvorgangs auftritt.

Eine bevorzugte Weiterbildung der Bearbeitungseinrichtung kann vorsehen, dass die Steuerungseinrichtung eine Positionserfassungseinrichtung aufweist, durch welche eine Position des Werkstücks zum Bearbeitungsaggregat ermittelbar ist.

Eine solche Positionserfassungseinrichtung kann beispielsweise durch eine dynamische Streckensteuerung ausgebildet sein. Diese kann über verschiedene Streckenpunkte eine exakte Position des Werkstücks auch bei unterschiedlichen Vorschubgeschwindigkeiten ermitteln. Ebenso kann die Positionserfassungseinrichtung durch einen oder mehrere optische oder taktile Sensoren ausgebildet sein. Durch die Positionsbestimmung kann eine präzise Ansteuerung der Stellbewegung des Bearbeitungsaggregats in Abhängigkeit zur Werkstückbewegung erfolgen.

### Kurze Beschreibung der Zeichnungen

Weitere Merkmale und Vorteile einer Vorrichtung, einer Verwendung und/oder eines Verfahrens ergeben sich aus der nachfolgenden Beschreibung von Ausführungsformen unter Bezugnahme auf die beiliegenden Zeichnungen. Von diesen Zeichnungen zeigt:
- Fig. 1: eine perspektivische Ansicht einer Ausführungsform einer Bearbeitungseinrichtung;
- Fig. 2: eine perspektivische Ansicht einer Ausführungsform eines offenbarungsgemäßen Bearbeitungsaggregats der Bearbeitungseinrichtung;
- Fig. 3: eine Seitenansicht des Bearbeitungsaggregats gemäß Figur 2;
- Fig. 4A: eine schematische Ansicht eines Tastelements des Bearbeitungsaggregats gemäß Figur 2 in einer Bereitschaftsposition;
- Fig. 4B: eine schematische Ansicht des Tastelements des Bearbeitungsaggregats gemäß Figur 2 in einer Tastposition;
- Fig. 5: eine perspektivische Ansicht einer weiteren Ausführungsform eines offenbarungsgemäßen Bearbeitungsaggregats der Bearbeitungseinrichtung;
- Fig. 6: eine Seitenansicht des Bearbeitungsaggregats gemäß Figur 5;
- Fig. 7A: eine schematische Ansicht eines Tastelements des Bearbeitungsaggregats gemäß Figur 5 in einer Bereitschaftsposition;
- Fig. 7B: eine schematische Ansicht des Tastelements des Bearbeitungsaggregats gemäß Figur 5 in einer Zwischenposition;
- Fig. 7C: eine schematische Ansicht des Tastelements des Bearbeitungsaggregats gemäß Figur 5 in einer Tastposition;

### Beschreibung von Ausführungsformen

Gleiche Bezugszeichen, die in verschiedenen Figuren aufgeführt sind, benennen identische, einander entsprechende, oder funktionell ähnliche Elemente.

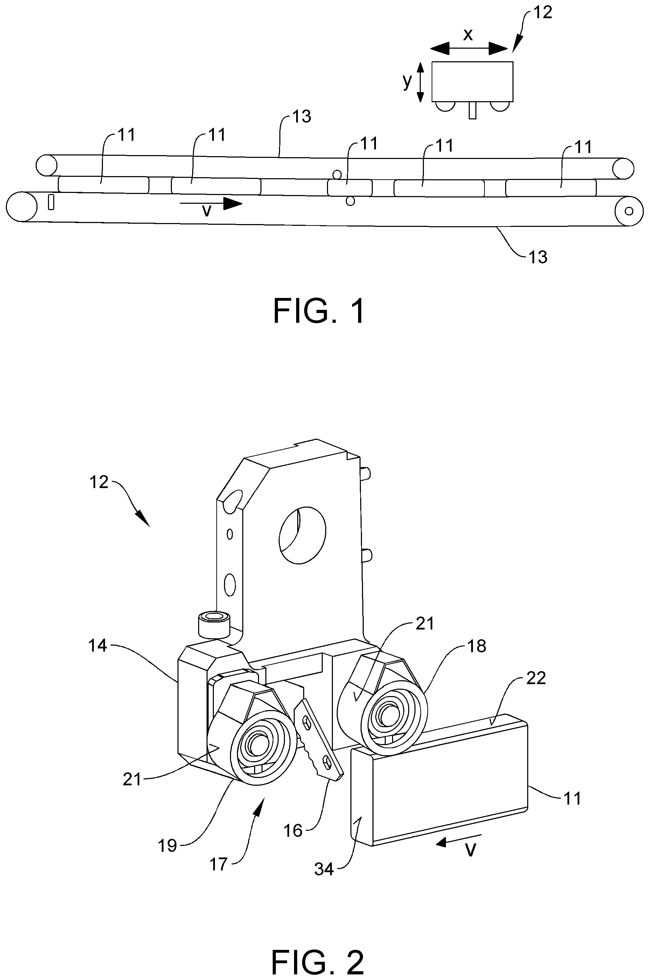

Figur 1 zeigt eine schematische Ansicht einer Bearbeitungseinrichtung 10. Vorzugsweise ist diese Bearbeitungseinrichtung 10 als eine Holzbearbeitungseinrichtung ausgebildet. Die Bearbeitungseinrichtung 10 kann als eine Durchlaufmaschine ausgebildet sein, bei welcher Werkstücke 11 für einen Bearbeitungsvorgang in einer Vorschubrichtung V relativ zu einem Bearbeitungsaggregat 12 der Bearbeitungseinrichtung 10 transportiert werden. Hierfür ist eine Transporteinrichtung 13 vorgesehen, welche die Werkstücke 11 im Durchlaufverfahren zum Bearbeitungsaggregat 12 transportiert. Ebenso kann die Bearbeitungseinrichtung 10 als eine stationäre Bearbeitungseinrichtung 10 ausgebildet sein, bei welcher die Werkstücke 11 stationär aufgenommen sind. Die Bearbeitungseinrichtung 10 kann als eine CNC-Maschine ausgebildet sein.

Die zu bearbeitenden Werkstücke 11 sind vorzugsweise zumindest teilweise aus Holz, Holzwerkstoffen, Kunststoff, einem Verbundwerkstoff oder dergleichen ausgebildet. Diese Werkstücke 11 können beispielsweise für die Möbel- oder Bauelementefertigung vorgesehen sein, z.B. in Form von Massivholzplatten, Spanplatten, Leichtbauplatten, Sandwichplatten, Profilleisten, Zierleisten oder dergleichen.

Figur 2 zeigt eine erste exemplarische Ausführungsform eines Bearbeitungsaggregats 12 der Bearbeitungseinrichtung 10. Die Bearbeitungseinrichtung 10 kann ein Bearbeitungsaggregat 12 oder mehrere Bearbeitungsaggregate 12 zum Bearbeiten der Werkstücke 11 aufweisen. Das Bearbeitungsaggregat 12 umfasst einen Grundkörper 14, an dem wenigstens ein Bearbeitungswerkzeug 16 zum Ausführen eines Bearbeitungsvorgangs sowie eine Tasteinrichtung 17 zum Positionieren des Bearbeitungsaggregats 12 zum zu bearbeitenden Werkstück 11 angeordnet sind.

Das Bearbeitungswerkzeug 16 kann ein beliebiges Werkzeug zum Ausführen eines Bearbeitungsvorgangs am Werkstück 11 sein. Beispielsweise kann das Bearbeitungswerkzeug 16 zum Bearbeiten einer Werkstückoberfläche, Werkstückkante oder eines am Werkstück 11 angebrachten Beschichtungsmaterials ausgebildet sein. Gemäß Figur 2 ist das Bearbeitungswerkzeug 16 als eine Profilziehklinge ausgebildet. Ebenso kann das Bearbeitungswerkzeug 16 ein Fräswerkzeug, Sägewerkzeug, Kappwerkzeug oder dergleichen sein.

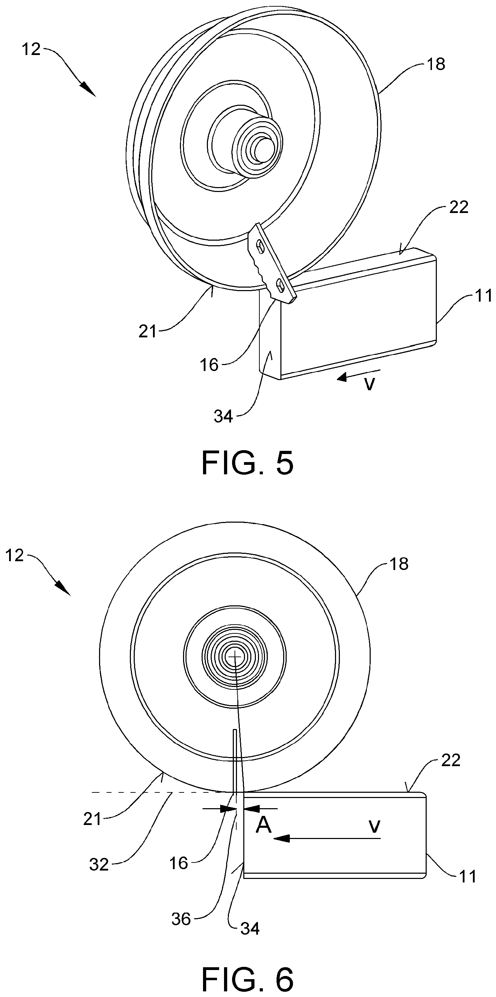

Durch die Tasteinrichtung 17 kann eine Werkstückoberfläche 22 abgetastet werden, sodass durch den Kontakt zum Werkstück 11 ein geführter Bearbeitungsvorgang durch das Bearbeitungswerkzeug 16 ausgeführt werden kann. Die Tasteinrichtung 17 gemäß Figur 2 umfasst ein erstes Tastelemente 18 sowie ein zweites Tastelement 19, wobei das Bearbeitungswerkzeug 16 zwischen den beiden Tastelementen 18, 19 angeordnet ist. Diese Tasteinrichtung 17 bildet somit eine Zwei-Rollen-Tastung. Zum Ausführen des Bearbeitungsvorgangs bzw. des Tastvorgangs wird zuerst das erste Tastelement 18 in Kontakt zu der Werkstückoberfläche 22 gebracht. Durch die Vorschubbewegung des Werkstücks 11 führt nachfolgend das Bearbeitungswerkzeug 16 den Bearbeitungsvorgang aus. Anschließend kommt durch die Vorschubbewegung des Werkstücks 11 das zweite Tastelement 19 in Kontakt zu der Werkstückoberfläche 22. Ebenso kann vorgesehen sein, dass die Tasteinrichtung 17 mehr als zwei Tastelemente 18, 19 aufweist. In einer zweiten exemplarischen Ausführungsform des Bearbeitungsaggregats 12, die nachfolgend bezüglich der Figuren 5 bis 7 beschrieben wird, kann die Tasteinrichtung 17 auch nur ein Tastelement 18 aufweisen. Die Tastelemente 18, 19 sind insbesondere als Tastrollen oder als Tastschuhe ausgebildet. Die Tastrollen bilden an deren Außenumfang eine Tastfläche 21, welche zum Abtasten einer Werkstückoberfläche 22 des Werkstücks 11 in Kontakt zum Werkstück 11 gebracht werden und mit welcher die Tastrollen auf der Werkstückoberfläche 22 abrollen.

Den Tastelementen 18, 19 kann eine nicht näher dargestellte Justiereinrichtung zugeordnet sein. Durch diese können die Tastelemente 18, 19 zum Bearbeitungswerkzeug 16 und/oder zum abzutastenden Werkstück 11 einstellbar bzw. justierbar sein. Dadurch können Werkstücktoleranzen ausgeglichen und/oder die Anpassung an unterschiedliche Geometrien oder Dimensionen von Werkstücken 11 ermöglicht sein.

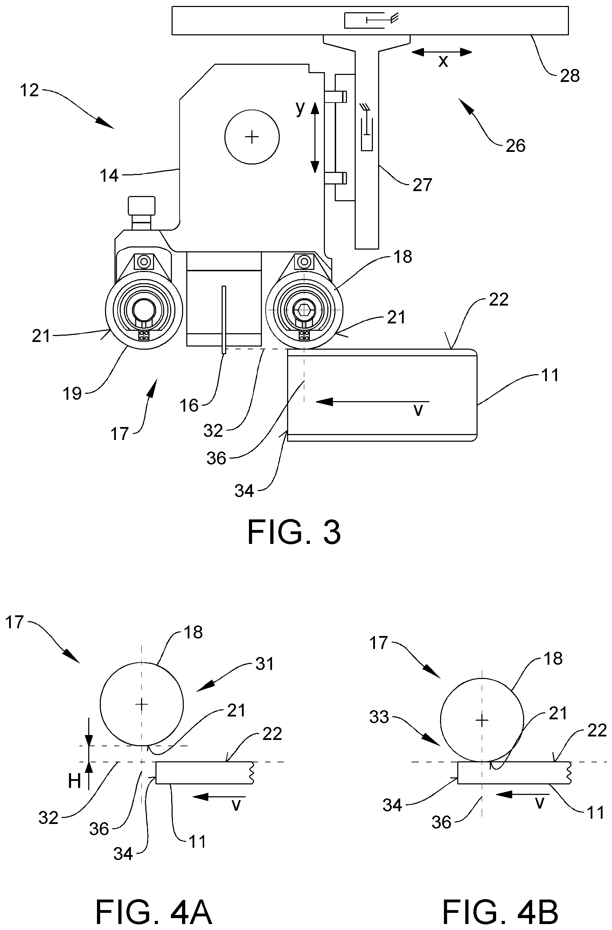

Figur 3 zeigt das Bearbeitungsaggregat 12 gemäß Figur 2 in einer Seitenansicht. Das Bearbeitungsaggregat 12 ist an einer Stelleinrichtung 26 angeordnet. Durch diese Stelleinrichtung 26 ist eine Stellbewegung des Bearbeitungsaggregats 12 ansteuerbar, um die Tasteinrichtung 17 sowie das Bearbeitungswerkzeug 16 für den Bearbeitungsvorgang zum Werkstück 11 zu positionieren. Durch die Stelleinrichtung 26 ist das Bearbeitungsaggregat 12 zwischen einer Bereitschaftsposition 31 und einer in Figur 3 dargestellten Tastposition 33 bewegbar, worauf nachfolgend näher eingegangen wird.

Durch die Stelleinrichtung 26 ist eine Stellbewegung des Bearbeitungsaggregats 12 ansteuerbar. Hierzu weist die Stelleinrichtung 26 eine erste Stelleinheit 27 auf, durch welche die Stellbewegung des Bearbeitungsaggregats 12 in eine Y-Richtung ansteuerbar ist. Zudem weist die Stelleinrichtung 26 eine zweite Stelleinheit 28 auf, durch welche die Stellbewegung des Bearbeitungsaggregats 12 in eine X-Richtung ansteuerbar ist. Auf diese Weise ist durch die Stelleinrichtung 26 eine mehrachsige Stellbewegung des Bearbeitungsaggregats 12 relativ zum Werkstück 12 in einer X-Y-Ebene ansteuerbar. Ebenso kann die Stelleinrichtung auch nur eine Stelleinheit 27 aufweisen durch welche die Stellbewegung in die X-Richtung oder eine Y-Richtung ansteuerbar ist. Zudem kann die Stelleinrichtung auch eine nicht näher dargestellte dritte Stelleinheit aufweisen, durch welche eine Stellbewegung in eine Z-Richtung ansteuerbar ist.

Zum Ansteuern der Stellbewegung weist die Stelleinrichtung 26 einen pneumatischen Antrieb, hydraulischen Antrieb oder einen elektrischen Antrieb auf. Dabei kann jeder Stelleinheit 27, 28 jeweils ein Pneumatik- oder Hydraulikzylinder oder ein Elektromotor zum Ansteuern der Stellbewegung zugeordnet sein. Zum Führen der Stellbewegung umfassen die Stelleinheiten 27, 28 jeweils eine Führung, beispielsweise eine Linearführung. Zudem weist die Bearbeitungseinrichtung 10 eine Steuerungseinrichtung auf, welche die Stellbewegung durch die Stelleinrichtung 26 steuert. Diese Steuerungseinrichtung kann in der Maschinensteuerung integriert sein.

Durch die Stelleinrichtung 26 ist ein präzises Positionieren des Bearbeitungsaggregats 12 zum Werkstück 11 ermöglicht, welches nachfolgend anhand der schematischen Abbildungen in den Figuren 4A und 4B erläutert wird. Dabei ist darauf hinzuweisen, dass die Figuren 4A und 4B aus Gründen der Übersichtlichkeit lediglich das gemäß Figur 2 und 3 erste Tastelement 18 des Bearbeitungsaggregats 12, welches durch die Stellbewegung zuerst in Kontakt zur Werkstückoberfläche 22 gebracht wird, zeigen und nicht maßstabsgetreu dargestellt sind.

Vor dem Kontakt mit dem Werkstück 11 ist das Bearbeitungsaggregat 12 in einer Bereitschaftsposition 31 angeordnet. In der Bereitschaftsposition 31 ist das Tastelement 18 in einem geringen Abstand zum Werkstück 11 angeordnet, wie in Figur 4A gezeigt. Dabei ist das Tastelement 18, insbesondere die Tastfläche 21 des Tastelements 18, oberhalb einer imaginären Werkstückebene 32, die in der abzutastenden Werkstückoberfläche 22 liegt, angeordnet.

Der Abstand zwischen der Tastfläche 21 des Tastelements 18 und der imaginären Werkstückebene 32 ist etwa 2,0 mm oder weniger. Insbesondere ist der Abstand etwa 1,0 mm oder weniger. Auf diese Weise ist ein Tasthub H aus der Bereitschaftsposition 31 in die Tastposition 33 ausgebildet, der einen Stellweg mit einer Länge von etwa 1,5 mm oder weniger, insbesondere etwa 1,0 mm oder weniger, aufweist.

Besonders bevorzugt beträgt die Länge des Tasthubs H etwa 0,7 mm oder weniger. Der Tasthub H kann auch nur wenige zehntel Millimeter betragen. Beispielsweise kann der Tasthub H eine Länge von etwa 0,1 mm bis 0,3 mm aufweisen. Dabei ist die Stellbewegung des Bearbeitungsaggregats 12 eine zur Werkstückoberfläche 22 gerichtete Stellbewegung. Insbesondere ist die Stellbewegung eine lineare Stellbewegung. Vorzugsweise wird die Stellbewegung im Wesentlichen orthogonal zur Werkstückoberfläche 22 ausgeführt. Ebenso kann die Stellbewegung als eine nichtlineare Stellbewegung ausgeführt werden. Hierzu kann die Stelleinrichtung 26 als eine Parallelogrammführung, eine Pendelführung oder eine Schwenkhebelführung ausgebildet sein.

Zwischen dem Bearbeitungsaggregat 12 und dem zu bearbeitenden Werkstück 11 wird eine Relativbewegung ausgeführt. Insbesondere wird das Werkstück 11 durch die Transporteinrichtung 13 in der Vorschubrichtung V zum Bearbeitungsaggregat 12 transportiert. Alternativ kann auch vorgesehen sein, dass das Bearbeitungsaggregat 12 relativ zum Werkstück 11 bewegt wird. Ebenso kann vorgesehen sein, dass das Werkstück 11 in Vorschubrichtung V transportiert wird und gleichzeitig das Bearbeitungsaggregat 12 relativ zum Werkstück 11 bewegt wird.

Um das erste Tastelement 18 in Kontakt mit der Werkstückoberfläche 22 zu bringen, d.h. dieses aus der Bereitschaftsposition 31 in die Tastposition 33 zu überführen, wird die Stellbewegung angesteuert, nachdem eine in Vorschubrichtung V gesehene Vorderkante 34 des Werkstücks 11 eine imaginäre Tastgrenze 36 passiert hat oder zu dem Zeitpunkt angesteuert, in dem die Vorderkante 34 des Werkstücks 11 die imaginäre Tastgrenze 36 erreicht. Die imaginäre Tastgrenze 36 verläuft durch die Mittelachse des Tastelements 18, d.h. die Mittelachse der zuerst auf die Werkstückoberfläche 22 aufsetzenden Tastrolle, und ist orthogonal zur Vorschubrichtung V des Werkstücks 11 ausgerichtet. Auf diese Weise wird das erste Tastelement 18 zum Abtasten des Werkstücks 11 mit der Tastfläche 21 durch einen sehr kurzen Tasthub H unmittelbar auf die Werkstückoberfläche 22 aufgesetzt.

Zum Ermitteln des Zeitpunkts zum Ausführen der Stellbewegung kann die Bearbeitungseinrichtung 10 eine nicht näher dargestellte Sensoreinrichtung aufweisen. Durch diese Sensoreinrichtung wird eine exakte Position des Werkstücks 11 zur Tasteinrichtung 17 bestimmt. Dies kann durch eine dynamische Streckensteuerung erfolgen, wobei über mehrere Streckenpunkte die Position des Werkstücks 11 berechnet wird. Ebenso kann die Sensoreinrichtung optische oder taktile Sensoren aufweisen, um die exakte Position des Werkstücks 11 zur Tasteinrichtung 17 zu ermitteln.

Die Figuren 5 und 6 zeigen eine zweite Ausführungsform des Bearbeitungsaggregats 12. Zur Vereinfachung ist das Bearbeitungsaggregat 12 in diesen Abbildungen lediglich durch das Bearbeitungswerkzeug 16 sowie das Tastelement 18 dargestellt. Im Gegensatz zu dem Bearbeitungsaggregat 12 gemäß der Figuren 2 und 3, weist die Tasteinrichtung 17 des Bearbeitungsaggregats 12 gemäß Figur 5 und 6 lediglich ein Tastelement 18 auf. Das Tastelement 18 ist als eine Tastrolle ausgebildet. Diese Tasteinrichtung 17 bildet somit eine EinRollen-Tastung. Der Durchmesser dieser Tastrolle ist größer als der der Tastrollen bei der Zwei-Rollen-Tastung gemäß Figur 2 und 3. Das Bearbeitungswerkzeug 16 ist etwa auf der Tastgrenze 36 oder nahe der Tastgrenze 36, die durch die Mittelachse der Tastrolle verläuft, angeordnet. Das Bearbeitungsaggregat 12 gemäß Figur 5 und 6 weist ebenfalls eine Stelleinrichtung 26 auf, wie diese bezüglich Figur 3 beschrieben ist die I. D.h., das Bearbeitungsaggregat 12 kann ebenfalls eine Stellbewegung in X-Richtung und/oder Y-Richtung ausführen.

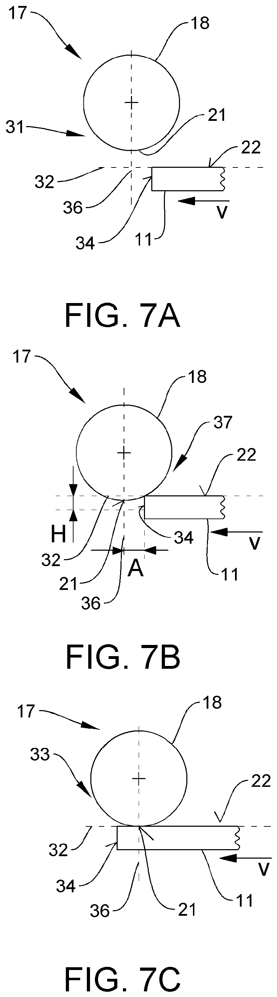

Nachfolgend wird anhand der Figuren 7A bis 7C die Stellbewegung des Bearbeitungsaggregats 12 zum Abtasten der Werkstückoberfläche 22 beschrieben.

Gemäß Figur 7A ist das Bearbeitungsaggregat 12 und somit auch das Tastelement 18 vor dem Kontakt mit dem Werkstück 11 in der Bereitschaftsposition 31 angeordnet. In der Bereitschaftsposition 31 ist das Tastelement 18 in einem geringen Abstand zum Werkstück 11 angeordnet, wobei das Tastelement 18, insbesondere die Tastfläche 21 des Tastelements 18, oberhalb der imaginären Tastgrenze 32, die in der abzutastenden Werkstückoberfläche 22 liegt, angeordnet ist. Der Abstand zwischen der Tastfläche 21 des Tastelements 18 und der imaginären Werkstückebene 32 ist etwa 1,5 mm oder weniger. Bevorzugt ist der Abstand etwa 1,0 mm oder weniger. Besonders bevorzugt ist der Abstand zwischen etwa 0,7 mm oder weniger.

Bei dieser Ausführungsform des Bearbeitungsaggregats 12 wird die Stellbewegung des Bearbeitungsaggregats 12 aus der Bereitschaftsposition 31 zunächst in eine in Figur 7B gezeigt Zwischenposition 37 angesteuert. Dabei wird die Stellbewegung angesteuert, bevor die in Vorschubrichtung V gesehene Vorderkante 34 des Werkstücks 11 die imaginäre Tastgrenze 36, die orthogonal zur Vorschubrichtung V des Werkstücks 11 ausgerichtet ist und die durch die Mittelachse des als Tastrolle ausgebildeten Tastelements 18 verläuft, passiert hat. Insbesondere wird die Stellbewegung in die Zwischenposition 37 ausgeführt, wenn die Werkstückvorderkante 34 in Vorschubrichtung V gesehen einen Abstand zur imaginären Tastgrenze 36 von etwa 10 mm oder weniger hat, vorzugsweise etwa 5 mm oder weniger oder 1 mm oder weniger.

In der Zwischenposition 37 ist die Tastfläche 21 des Tastelements 18 in Bezug zum Werkstück 11 unterhalb der imaginären Werkstückebene 32 angeordnet. Auf diese Weise wird das Tastelement 18 nicht unmittelbar auf die abzutastende Werkstückoberfläche 22 aufgesetzt, sondern durch dass sich in Vorschubrichtung V bewegende Werkstück 11 durch einen Tasthub H in die Tastposition 33 gedrückt. Dabei ist vorgesehen, dass der Tasthub H aus der Zwischenposition 37 in die Tastposition 33 einen Hubweg von weniger als 1,0 mm, vorzugsweise weniger als etwa 0,5 mm, aufweist. Besonders bevorzugt ist vorgesehen, dass der Tasthub H nur wenige zehntel Millimeter aufweist. Der Tasthub H kann eine Länge von etwa 0,1 mm bis 0,3 mm aufweisen.

Dadurch führt das Tastelement 18 einen sehr kurzen Tasthub H aus, um in die Tastposition 33 überführt zu werden. Auf diese Weise erfährt die Tasteinrichtung 17 durch den Kontakt mit dem Werkstück 11 nur einen geringen Stoßimpuls, sodass das Bearbeitungsaggregat 12 in keinen bzw. keinen nennenswerten Schwingungszustand versetzt wird. Dadurch wird die Bearbeitungsqualität durch das Bearbeitungswerkzeug 16 am Werkstück 11 insbesondere im Anfangsbereich des Bearbeitungsvorgangs signifikant verbessert.

## Patentansprüche

1. Verfahren zum Positionieren eines Bearbeitungsaggregats (12) zu einem zu bearbeitenden Werkstück (11), das bevorzugt zumindest teilweise aus Holz, Holzwerkstoffen, Kunststoff, Verbundwerkstoff oder dergleichen ausgebildet ist, wobei zwischen dem Bearbeitungsaggregat (12) und dem zu bearbeitenden Werkstück (11) eine Relativbewegung ausgeführt wird, eine Tasteinrichtung (17) des Bearbeitungsaggregats (12) zum Abtasten einer Werkstückoberfläche (22) in Kontakt mit dem Werkstück (11) gebracht wird und mittels der Tasteinrichtung (17) ein geführter Bearbeitungsvorgang durch ein Bearbeitungswerkzeug (16) des Bearbeitungsaggregats (12) ausgeführt wird, **dadurch gekennzeichnet, dass** das Bearbeitungsaggregat (12) vor dem Kontakt mit dem Werkstück (11) in einer Bereitschaftsposition (31) angeordnet wird, in der wenigstens ein Tastelement (18, 19) der Tasteinrichtung (17) in Bezug zum Werkstück (11) oberhalb oder unterhalb einer imaginären Werkstückebene (32), die in der abzutastenden Werkstückoberfläche (22) liegt, angeordnet ist, und das wenigstens eine Tastelement (18, 19) in die Tastposition (33) oder eine Zwischenposition (37) überführt wird, indem durch eine Stelleinrichtung (26) eine zur imaginären Werkstückebene (32) gerichtete Stellbewegung des Bearbeitungsaggregats (12) angesteuert wird.

2. Verfahren nach Anspruch 1, bei dem ferner die Stellbewegung des Bearbeitungsaggregats (12) aus der Bereitschaftsposition (31) in die Tastposition (33) oder die Zwischenposition (37) einen Stellweg aufweist, der eine Länge von etwa 2,0 mm oder kürzer, vorzugsweise etwa 1,0 mm oder kürzer, aufweist.

3. Verfahren nach Anspruch 1 oder 2, bei dem ferner die Stellbewegung des Bearbeitungsaggregats (12) als eine lineare oder nichtlineare Stellbewegung ausgeführt wird.

4. Verfahren nach einem der vorhergehenden Ansprüche, bei dem ferner die Stellbewegung des Bearbeitungsaggregats (12) im Wesentlichen orthogonal zur abzutastenden Werkstückoberfläche (22) ausgeführt wird.

5. Verfahren nach einem der vorhergehenden Ansprüche, bei dem ferner ein als Tastrolle ausgebildetes Tastelement (18) mit einer Tastfläche (21) auf die Werkstückoberfläche (22) aufgesetzt wird, indem die Stellbewegung des Bearbeitungsaggregats (12) aus der Bereitschaftsposition (31) in die Tastposition (33) angesteuert wird, nachdem eine in Vorschubrichtung (V) gesehene Vorderkante (34) des Werkstücks (11) eine imaginäre Tastgrenze (36), die durch die Mittelachse der Tastrolle verläuft und die orthogonal zur Werkstückoberfläche (22) ausgerichtet ist, passiert hat oder die Stellbewegung zum Zeitpunkt des Passierens der imaginären Tastgrenze (36) ausgeführt wird.

6. Verfahren nach einem der Ansprüche 1 bis 4, bei dem ferner die Stellbewegung des Bearbeitungsaggregats (12) aus der Bereitschaftsposition (31) in die Zwischenposition (37) angesteuert wird, bevor eine in Vorschubrichtung (V) gesehene Vorderkante (34) des Werkstücks (11) eine imaginäre Tastgrenze (36), die durch die Mittelachse eines als Tastrolle ausgebildeten Tastelements (18) verläuft und die orthogonal zur Werkstückoberfläche (22) ausgerichtet ist, passiert hat, wobei eine Tastfläche (21) des Tastelements (18) in der Zwischenposition (37) in Bezug zum Werkstück (11) unterhalb der imaginären Werkstückebene (32) angeordnet ist und das Tastelement (18) durch die Vorschubbewegung (V) des Werkstücks (11) durch einen Tasthub (H) in die Tastposition (33) gedrückt wird.

7. Verfahren nach Anspruch 6, bei dem ferner der Tasthub (H) aus der Zwischenposition (37) in die Tastposition (33) einen Hubweg von weniger als etwa 1,0 mm, vorzugsweise weniger als etwa 0,5 mm, aufweist.

8. Verfahren nach Anspruch 6 oder 7, bei dem ferner die Stellbewegung in die Zwischenposition (37) ausgeführt wird, wenn der Abstand der Werkstückvorderkante (34) des sich in Vorschubrichtung (V) bewegenden Werkstücks (11) zur imaginären Tastgrenze (36) etwa 10 mm oder weniger ist, vorzugsweise etwa 5 mm oder weniger.

9. Bearbeitungseinrichtung (10) zum Bearbeiten von Werkstücken (11), die bevorzugt zumindest teilweise aus Holz, Holzwerkstoffen, Kunststoff, Verbundwerkstoff oder dergleichen ausgebildet sind, mit einem Bearbeitungsaggregat (12) zum Ausführen eines Bearbeitungsvorgangs, einer Fördereinrichtung (13) zum Herbeiführen einer Relativbewegung zwischen dem Bearbeitungsaggregat (12) und dem zu bearbeitenden Werkstück (11) sowie mit einer Tasteinrichtung (17) zum Führen des Bearbeitungsaggregats (12) während des Bearbeitungsvorgangs, **dadurch gekennzeichnet, dass** das Bearbeitungsaggregat (12) eine Stelleinrichtung (26) aufweist und eine Steuerungseinrichtung vorgesehen ist, die zur Ausführung eines Verfahrens nach einem der Ansprüche 1 bis 8 konfiguriert ist.

10. Bearbeitungseinrichtung nach Anspruch 9, bei dem ferner die Steuerungseinrichtung eine Positionserfassungseinrichtung aufweist, durch welche eine Position des Werkstücks (11) zum Bearbeitungsaggregat (12) ermittelbar ist.

## Claims

1. Method for positioning a machining unit (12) relative to a workpiece (11) to be machined, which workpiece is preferably made at least in part of wood, wood-based materials, plastics material, composite material or the like, wherein a relative movement is performed between the machining unit (12) and the workpiece (11) to be machined, a tracing device (17) of the machining unit (12) for tracing a workpiece surface (22) is brought into contact with the workpiece (11) and a guided machining process by a machining tool (16) of the machining unit (12) is performed by means of the tracing device (17), **characterised in that** the machining unit (12) is arranged in a standby position (31) prior to contact with the workpiece (11), in which standby position at least one tracing element (18, 19) of the tracing device (17) is arranged above or below an imaginary workpiece plane (32) in relation to the workpiece (11), which plane lies in the workpiece surface (22) to be traced, and the at least one tracing element (18, 19) is transferred into the tracing position (33) or an intermediate position (37) by an adjustment movement of the machining unit (12) that is directed towards the imaginary workpiece plane (32) being triggered by an adjustment device (26).

2. Method according to claim 1, wherein the adjustment movement of the machining unit (12) from the standby position (31) into the tracing position (33) or intermediate position (37) has an adjustment range that has a length of approximately 2.0 mm or less, preferably approximately 1.0 mm or less.

3. Method according to claim 1 or 2, wherein the adjustment movement of the machining unit (12) is further performed as a linear or non-linear adjustment movement.

4. Method according to any of the preceding claims, wherein the adjustment movement of the machining unit (12) is further performed substantially orthogonally to the workpiece surface (22) to be traced.

5. Method according to any of the preceding claims, wherein a tracing element (18) designed as a tracing roller and having a tracing surface (21) is placed onto the workpiece surface (22) by the adjustment movement of the machining unit (12) from the standby position (31) into the tracing position (33) being triggered after a front edge (34), viewed in the feed direction (V), of the workpiece (11) has passed through an imaginary tracing boundary (36) that extends through the centre axis of the tracing roller and that is oriented orthogonally to the workpiece surface (22), or by the adjustment movement being performed at the point in time at which the imaginary tracing boundary (36) is passed.

6. Method according to any of claims 1 to 4, wherein the adjustment movement of the machining unit (12) from the standby position (31) into the intermediate position (37) is further triggered before a front edge (34), viewed in the feed direction (V), of the workpiece (11) has passed through an imaginary tracing boundary (36) that extends through the centre axis of a tracing element (18) designed as a tracing roller and that is oriented orthogonally to the workpiece surface (22), wherein a tracing surface (21) of the tracing element (18) is arranged below the imaginary workpiece plane (32) in relation to the workpiece in the intermediate position (37) and the tracing element (18) is pushed by a tracing stroke (H) into the tracing position (33) by the feed movement (V) of the workpiece (11).

7. Method according to claim 6, wherein the tracing stroke (H) from the intermediate position (37) into the tracing position (33) further has a stroke range of less than approximately 1.0 mm, preferably less than approximately 0.5 mm.

8. Method according to claim 6 or 7, wherein the adjustment movement into the intermediate position (37) is further performed when the distance between the workpiece front edge (34) of the workpiece (11) moving in the feed direction (V) and the imaginary tracing boundary (36) is approximately 10 mm or less, preferably approximately 5 mm or less.

9. Machining device (10) for machining workpieces (11) that are preferably made at least in part of wood, wood-based materials, plastics material, composite material or the like, comprising a machining unit (12) for performing a machining process, a conveying device (13) for bringing about a relative movement between the machining unit (12) and the workpiece (11) to be machined and comprising a tracing device (17) for guiding the machining unit (12) during the machining process, **characterised in that** the machining unit (12) has an adjustment device (26), and a control device is provided which is configured to perform a method according to any of claims 1 to 8.

10. Machining device according to claim 9, wherein the control device further has a position detection device by means of which a position of the workpiece (11) relative to the machining unit (12) can be determined.

## Revendications

1. Procédé de positionnement d'une unité d'usinage (12) par rapport à une pièce (11) à usiner, qui est de préférence réalisée au moins partiellement en bois, en des matériaux dérivés du bois, de matière plastique, de matériaux composites ou similaires, dans lequel un mouvement relatif est exécuté entre l'unité d'usinage (12) et la pièce (11) à usiner, un dispositif (17) palpeur de l'unité d'usinage (12) est mise en contact avec la pièce (11) pour palper une surface (22) de pièce et un processus d'usinage guidé est exécuté par un outil (16) d'usinage de l'unité d'usinage (12) au moyen du dispositif (17) palpeur, **caractérisé en ce que** l'unité d'usinage (12) avant le contact avec la pièce (11) est placée dans une position de disponibilité (31), dans laquelle au moins un élément (18, 19) palpeur du dispositif (17) palpeur est placé par rapport à la pièce (11) au-dessus ou en dessous d'un plan (32) de pièce imaginaire qui se trouve dans la surface (22) de pièce à palper, et **en ce que** ledit au moins un élément (18, 19) palpeur est amené dans la position (33) de palpage ou dans une position intermédiaire (37) du fait qu'un mouvement de réglage de l'unité d'usinage (12) dirigé vers le plan (32) de pièce imaginaire est commandé par un dispositif de réglage (26).

2. Procédé selon la revendication 1, selon lequel en outre le mouvement de réglage de l'unité d'usinage (12) à partir de la position de disponibilité (31) dans la position (33) de palpage ou la position intermédiaire (37) présente une course de réglage qui présente une longueur d'environ 2,0 mm ou moins, de préférence d'environ 1,0 mm ou moins.

3. Procédé selon la revendication 1 ou la revendication 2, selon lequel en outre le mouvement de réglage de l'unité d'usinage (12) est exécuté comme un mouvement de réglage linéaire ou non linéaire.

4. Procédé selon l'une quelconque des revendications précédentes, selon lequel en outre le mouvement de réglage de l'unité d'usinage (12) est exécuté sensiblement orthogonalement par rapport à la surface (22) de pièce à palper.

5. Procédé selon l'une quelconque des revendications précédentes, selon lequel en outre un élément (18) palpeur réalisé comme un galet palpeur avec une surface (21) de palpage est posé sur la surface (22) de pièce, du fait que le mouvement de réglage de l'unité d'usinage (12) à partir de la position de disponibilité (31) dans la position (33) de palpage est commandé, après qu'un bord avant (34) de la pièce (11) vu dans la direction (V) d'avance a franchi une limite (36) de palpage imaginaire, qui passe par l'axe central du galet palpeur et est orienté orthogonalement par rapport à la surface (22) de pièce, ou le mouvement de réglage est exécuté au moment du franchissement de la limite (36) de palpage imaginaire.

6. Procédé selon l'une quelconque des revendications 1 à 4, selon lequel en outre le mouvement de réglage de l'unité d'usinage (12) à partir de la position de disponibilité (31) dans la position intermédiaire (37) est en outre commandé avant qu'un bord avant (34) de la pièce (11) vu dans la direction (V) d'avance ait franchi une limite (36) de palpage imaginaire, qui passe par l'axe central d'un élément (18) palpeur réalisé comme un galet palpeur et qui est orienté orthogonalement par rapport à la surface (22) de pièce, dans lequel une surface (21) de palpage de l'élément (18) palpeur est disposée dans la position intermédiaire (37) par rapport à la pièce (11) en dessous du plan (32) de pièce imaginaire et l'élément (18) palpeur est pressé par le mouvement d'avance (V) de la pièce (11) par une course (H) de palpage dans la position (33) de palpage.

7. Procédé selon la revendication 6, selon lequel en outre la course (H) de palpage à partir de la position intermédiaire (37) dans la position (33) de palpage présente une course de levage inférieure à environ 1,0 mm, de préférence inférieure à environ 0,5 mm.

8. Procédé selon la revendication 6 ou la revendication 7, selon lequel en outre le mouvement de réglage dans la position intermédiaire (37) est exécuté lorsque l'écart du bord avant (34) de pièce de la pièce (11) se déplaçant dans la direction (V) d'avance par rapport à la limite (36) de palpage imaginaire est d'environ 10 mm ou moins, de préférence d'environ 5 mm ou moins.

9. Dispositif (10) d'usinage pour usiner des pièces (11) qui sont de préférence réalisées au moins partiellement en bois, en matériaux dérivés du bois, en matière plastique, en matériaux composites ou similaires, comprenant une unité d'usinage (12) pour exécuter une opération d'usinage, un dispositif de transport (13) pour provoquer un mouvement relatif entre l'unité d'usinage (12) et la pièce (11) à usiner ainsi qu'un dispositif (17) palpeur pour guider l'unité d'usinage (12) pendant l'opération d'usinage, **caractérisé en ce que** l'unité d'usinage (12) présente un dispositif de réglage (26) et **en ce qu'**un dispositif de commande est prévu, qui est conçu pour mettre en œuvre un procédé selon l'une quelconque des revendications 1 à 8.

10. Dispositif d'usinage selon la revendication 9, pour lequel en outre le dispositif de commande présente un dispositif de détection de position par lequel une position de la pièce (11) par rapport à l'unité d'usinage (12) peut être déterminée.
